(19) Europäisches Patentamt

European Patent Office

Office européen des brevets

(11) **EP 0 756 480 B1**

(12) **EUROPÄISCHE PATENTSCHRIFT**

(45) Veröffentlichungstag und Bekanntmachung des Hinweises auf die Patenterteilung:
**29.08.2001 Patentblatt 2001/35**

(21) Anmeldenummer: **95915844.5**

(22) Anmeldetag: **05.04.1995**

(51) Int Cl.[7]: **A61K 9/16**

(86) Internationale Anmeldenummer:
**PCT/EP95/01236**

(87) Internationale Veröffentlichungsnummer:
**WO 95/28147 (26.10.1995 Gazette 1995/46)**

(54) **RETARD-MATRIXPELLETS UND VERFAHREN ZU IHRER HERSTELLUNG**

SUSTAINED-RELEASE MATRIX PELLETS AND METHOD FOR PREPARING THEM

PELLETS A LIBERATION RETARD A MATRICE D'ENROBAGE ET LEUR PROCEDE DE PREPARATION

(84) Benannte Vertragsstaaten:
**AT BE CH DE DK ES FR GB IT LI LU NL PT SE**

(30) Priorität: **18.04.1994 DE 4413350**

(43) Veröffentlichungstag der Anmeldung:
**05.02.1997 Patentblatt 1997/06**

(73) Patentinhaber: **BASF AKTIENGESELLSCHAFT 67056 Ludwigshafen (DE)**

(72) Erfinder:
• **GRABOWSKI, Sven 67061 Ludwigshafen (DE)**
• **ROSENBERG, Joerg 67158 Ellerstadt (DE)**
• **SANNER, Axel 67227 Frankenthal (DE)**

(56) Entgegenhaltungen:
**EP-A- 0 095 123       EP-A- 0 204 596
EP-A- 0 388 954       EP-A- 0 544 144
WO-A-93/07859**

• **CHEMICAL & PHARMACEUTICAL BULLETIN, Bd. 39, Nr. 2, Februar 1991 TOKYO (JP), Seiten 465-467, H. YUASA ET AL. 'application of the solid dispersion method to controlled release of medicine. I. controlled release of water soluble medicine by using solid dispersion'**

**Beschreibung**

Die Erfindung betrifft eine feste, vorzugsweise pharmazeutische, Retardform (Pellets), bei der der Wirkstoff in eine Mischung von einem wasserunlöslichen Polymeren, einem Lipid sowie einem kolloidal hochviskos wasserlöslichen, gelbildenden oder zumindest wasserquellbaren Polymeren eingebettet ist. Die Herstellung erfolgt in einem einstufigen, kontinuierlichen Prozeß durch Schmelzextrusion und Formgebung, vorzugsweise durch Heißabschlag.

Stand der Technik

Für die Schmelz-Extrusion und Retardierung geeignete Matrixsubstanzen sind unter Druck und Temperatur plastifizierbare Polymere und Lipide. Speiser et al. beschreiben in Pharm. Acta Helv. <u>46</u>, 31 (1971) den Einsatz von magensaftlöslichen Epoxid-Aminharzen sowie darmsaftlöslichen Copolymeren aus Vinylacetat/Crotonsäure für spritzgegossene Arzneiformen (vgl. hierzu das zu US 3,432,592 Gesagte). Hüttenrauch und Schmeiss untersuchten die Freisetzung von Modellwirkstoffen aus einer durch Kolbenextrusion hergestellten Polyethylen-Matrix (Pharmazie <u>30</u>, 229, 536 (1975)).

Mank et al. beschreiben in Pharmazie <u>44</u>, 773 (1989) und ibid. <u>45</u>, 592 (1990) die Wirkstoff-Freisetzung aus unlöslichen Thermoplast-Matrices. Diese Methoden erlauben keine frei einstellbare Retardierung, und der Wirkstoff wird insbesondere aus den Polyethylen-Matrizes nicht vollständig freigesetzt. Hinzu kommen bei diesem Verfahren die Nachteile des Spritzgusses wie lange Verweilzeit bei hoher Temperatur und hohe Materialverluste durch die Angußkanäle, deren Inhalt keiner Wiederverwendung zugeführt werden darf. Außerdem sind die Formkosten relativ zur Produktionsgeschwindigkeit außerordentlich hoch.

N.A. El Gindy et al. beschreiben in Acta Pharm. Technol. <u>33</u>, 208-211 (1987) die Herstellung von Tabletten durch Schmelzen von Mischungen von Wirkstoffen mit wasserlöslichen (Polyethylenglykole und Polyoxyethylen-polyoxypropylen-Blockcopolymeren) und unlöslichen Polymeren und anschließendem Pressen. Aufgrund der Polymerwahl sind diese Formen mehr oder weniger schnellfreisetzend. Das Herstellverfahren ist diskontinuierlich.

N. Follonier et al. berichten in Capsule News 1 (1991), 2 und im Abstract der 6th International Conference on Pharmaceutical Technology, Paris, France, 2. bis 4. Juni 1992, über die Herstellung von Sustained-Release-Pellets durch Schmelzextrusion aus einem Ein-Schnecken-Extruder. Das erstarrte Extrudat zerkleinerte man in einem Messer-Pelletizer. Als Matrix setzte man wasserunlösliche Polymere ein. Neben der Pelletgröße wurden verschiedene Zusätze zur Steuerung der Wirkstoff-Freisetzung untersucht. Die Polymerbasis war hauptsächlich ein Ethylen-Vinylacetat-Copolymer. Eine Freisetzung des Wirkstoffs aus diesen Formen nach "Nullter-Ordnung-Kinetik" gelang jedoch nicht.

US 3,432,592 beschreibt den Spritzguß von wirkstoffhaltigen Polymerschmelzen. Die dort verwendeten Polymere sollen in den Verdauungssäften mindestens teilweise löslich sein. Als lösliches Polymer wird überwiegend ein im Pharmabereich nicht übliches komplexes Kondensationsprodukt aus einem Aminodiol und einem Epoxid beschrieben. Eine Retardierung wird erreicht durch die Mitverwendung von in den Verdauungssäften schwer löslichen Thermoplasten. Die dort angegebenen Polymerkombinationen sind nicht zur Retardierung gut wasserlöslicher Wirkstoffe aus Pellets geeignet aufgrund des ungünstigen Oberflächen/Volumen-Verhältnisses. Generell läßt sich die Retardierung nach dieser Arbeitsweise schlecht steuern, bei starker Retardierung verbleibt ein Teil des Wirkstoffs ungelöst in den Pellets (Freisetzung des Wirkstoffs nach $\sqrt{t}$-Gesetz; s. T. Higuchi, J. Pharm. Sci. <u>52</u>, 1145-1149 (1963)). Eine Freisetzung nach "Nullter-Ordnung-Kinetik" gelingt nicht (vgl. Tabelle I).

Das Extrudieren von wirkstoffhaltigen Polymerschmelzen, vorzugsweise von Vinylpyrrolidon-Copolymeren, ist aus EP-A 240 904 und EP-A 240 906 bekannt. Die Einstellung eines bestimmten Wirkstoff-Freisetzungsprofils über Polymermischungen ist dort nicht ausgeführt. Darüber hinaus hat sich gezeigt, daß die so hergestellten Produkte in vielen Fällen wenig lagerbeständig sind, der Retardeffekt nimmt mit der Zeit ab.

EP-B 204 596 beschreibt die Herstellung von Pellets durch Einbettung eines Wirkstoffes in eine Matrix aus folgenden Komponenten: Mindestens einem nichthydrophilen Polymer und entweder einer Mischung von mindestens zwei Lipid-Stoffen, wovon der eine das oder die Polymere lösende oder gelierende Eigenschaften und der andere Schmiermitteleigenschaften besitzt, oder einem Lipid-Stoff, der die beiden genannten Eigenschaften in sich vereinigt, und gegebenenfalls einem oder mehreren Zusätzen, ausgewählt aus Streckmitteln und antistatischen Mitteln. Gravierende Nachteile: Bei höheren Mengen (ab ca. 20%) an "nicht hydrophilem Polymer" erfolgt die Freisetzung für ein Retardpräparat zu rasch, bei geringeren Mengen ändert sich die Freisetzung stark beim Lagern, und sie ist unvollständig.

In der EP-A 544 144 sind feste pharmazeutische Retardformen beschrieben, die durch Schmelzextrusion von Mischungen eines pharmazeutischen Wirkstoffs mit einem Polymerengemisch erhalten werden, wobei das Polymerengemisch mindestens ein wasserunlösliches Pol(meth)acrylat sowie ein wasserlösliches Hydroxyalkylcellulosederivat oder ein N-Vinylpyrrolidonpolymerisat oder Mischungen davon enthält.

Der Erfindung lag die Aufgabe zugrunde, Pellets, vorzugsweise für pharmazeutische Zwecke, herzustellen, aus denen der Wirkstoff mit einstellbarem Retardprofil, also beliebig verzögert - aber vollständig -,freigesetzt wird.

Dieses Ziel sollte durch Matrixpellets realisiert werden, also ohne auf den Pelletkern aufgebrachte retardierende Filmüberzüge.

Neben der Steuerung der Wirkstoff-Freisetzung durch die Zusammensetzung der Matrix (Matrix-Retardpellets) sollte eine Technik zur einfachen kostengünstigen Herstellung dieser Pellets entwickelt werden. Dieses Verfahren sollte kontinuierlich und einstufig sein ohne vorhergehende Mischung oder Vor-Granulation der Komponenten und ohne abschließende Spheronisation oder ähnliche Ausformung/ Abrundung der Pellets nach dem Herstellungsprozeß.

Lösung

Es wurde nun gefunden, daß es durch Schmelzextrusion bestimmter wirkstoffhaltiger Polymermatrices und anschließende kontinuierliche Formgebung in einfacher Weise gelingt, Retardpellets mit hohen Wirkstoffgehalten, auch von sehr gut wasserlöslichen Wirkstoffen, herzustellen, wobei sich allein durch die Zusammensetzung der Polymermatrix ohne diffusionskontrollierende Polymerüberzüge über weite Bereiche einstellbare Freisetzungsprofile mit hoher Lagerbeständigkeit erzielen lassen.

Das Grundprinzip der erfindungsgemäßen Polymermatrix ist eine durch geeignete lipophile Substanzen plastifizierte Matrix aus einem in Wasser und gastrointestinalen Flüssigkeiten unlöslichen Polymeren. Im Gegensatz zu dem oben angeführten Stand der Technik gelingt nun ein über weite Bereiche frei einstellbares Retardprofil, wenn zusätzlich in die Matrix aus unlöslichem Polymer und lipophiler Komponente ein Gelbildner, also ein in Wasser hochviskos lösliches (Hydrokolloid) oder zumindest quellbares Polymer eingebaut wird. Bei den oben im Stand der Technik genannten Matrices wird zwar die Freisetzung des Wirkstoffs über die Konzentration an unlöslichem Polymer kontrolliert, bei einer zu geringen Menge an Polymer besteht aber die Gefahr, daß die Darreichungsform zerfällt, bei einer zu großen Menge an Polymer kann jedoch die Wirkstoff-Freisetzung unvollständig werden, Anteile des Wirkstoffs werden komplett eingeschlossen und sind nicht verfügbar. Durch den erfindungsgemäßen Zusatz an Gelbildner wird ein "Aufbrechen" der Retardmatrix durch Quellung dieses Polymeren erreicht, der Wirkstoff kann vollständig freigesetzt werden (vgl. Tab. I).

Die erfindungsgemäße Polymermatrix für die Matrix-Retardpellets ist eine neue Kombination aus inerter, lipophiler und hydrophiler Matrix, thermoplastisch verarbeitbar.

Erfindungsgegenstand ist daher eine feste pharmazeutische Retardform (Matrixpellets), hergestellt in einem einstufigen Verfahren durch Schmelzextrusion in einem Extruder, vorzugsweise einem Zwei-Schnecken-Extruder oder einem Ein-Schnecken-Extruder mit Mischabteil, bei 50 bis 200°C und kontinuierliche Formgebung (vorzugsweise Heißabschlag) einer Mischung folgender Zusammensetzung:

a) mindestens eine biologisch wirksame Verbindung ("Wirkstoff"; vorzugsweise human- oder veterinär-med., aber auch Vitamine sowie systemische Insektizide, Fungizide und Herbizide) in einer Menge von 0,1 bis 87, vorzugsweise 1 bis 75, insbesondere 45 bis 75 Gew.-%,

b) mindestens ein in Wasser und gastrointestinalen Flüssigkeiten unlösliches natürliches, halbsynthetisches oder synthetisches Polymer in einer Menge von 5 bis 50, vorzugsweise 10 bis 40 Gew.-%,

c) 5 bis 45, vorzugsweise 10 bis 35 Gew.-% mindestens einer wasserunlöslichen liphophilen Komponente mit Weichmachereigenschaften bezüglich des Polymeren b) und Gleit- bzw. Schmiermitteleigenschaften,

d) 3 bis 40, vorzugsweise 5 bis 25 Gew.-% mindestens eines in Wasser oder gastrointestinalen Flüssigkeiten kolloidal löslichen, hochviskose Lösungen oder Gele bildenden oder mindestens quellbaren natrülichen oder halbsynthetischen hydrophilen Polymeren (hier kurz "Gelbildner" genannt), und

e) 0 bis 50, vorzugsweise 0 bis 40 Gew.-% eines oder mehrerer der üblichen Formulierungshilfsstoffe.

Die Prozentangaben beziehen sich jeweils auf das Gesamtgewicht der Pellets.

Feste pharmazeutische Retardformen im Sinne der Erfindung sind z.B. Granulate, vorzugsweise Pellets, mit verzögerter Wirkstoff-Freisetzung. Die erhaltenen Formlinge können auch anschließend zu Pulver gemahlen und in dieser Form eingesetzt werden (z.B. in Hartgelatine-Kapseln). Das nachträgliche Überziehen der Formlinge mit geschmackabdeckenden Filmüberzügen, wie sie Stand der Technik sind (z.B. mit Polyacrylaten, Celluloseestern wie Hydroxypropylmethylcellulosephthalaten und Celluloseethern wie Ethylcellulose, Hydroxypropylmethylcellulose oder Hydroxypropylcellulose), wird nicht ausgeschlossen, ist aber in aller Regel nicht erforderlich.

Ein Verpressen der Pellets zu Tabletten ist in vielen Fällen möglich. Dieses Verpressen bietet sich u.a. in den Fällen an, wo die Wirkstoffdosis hoch ist und somit unerwünscht große Arzneiformen resultieren würden. Durch Steuerung der Tablettierbedingungen (insbesondere Preßdruck) können u.U. einzelne Pellets zerfallen, so daß die Wirkstoffreigabe nicht wesentlich von analogen Pellets verschieden sein muß, die (lose) in Kapseln abgefüllt wurden. Die Verpressung zu Tabletten führt zu einer Volumenreduktion der Arzneiform, die in Einzelfällen vorteilhaft sein kann. Durch Zugabe osmotisch wirksamer Agenzien (z.B. anorganische Salze) können ferner Pellets erhalten werden, die als osmotisch aktive Quellschicht eingesetzt werden können (vgl. WO 92/04011), um die Wirkstoffabgabe z.B. aus Tabletten (nach Verpressen) oder Kapseln über ein Osmoseprinzip zu bewirken.

Unter pharmazeutischen Wirkstoffen a) im Sinne der Erfindung sind alle Stoffe mit einer pharmazeutischen Wirkung und möglichst geringen Nebenwirkungen zu verstehen, sofern sie sich unter den Verarbeitungsbedingungen nicht zersetzen. Die Wirkstoffmenge pro Dosis-Einheit und die Konzentration können je nach Wirksamkeit und gewünschter Freisetzungsgeschwindigkeit in weiten Grenzen variieren. Die einzige Bedingung ist, daß sie zur Erzielung der gewünschten Wirkung ausreichen. So kann die Wirkstoffkonzentration im Bereich von 0,1 bis 87, vorzugsweise 1 bis 80, insbesondere 45 bis 75 Gew.-% liegen. Wirkstoffe im Sinne der Erfindung sind auch, wie erwähnt, andere biologisch wirksame Verbindungen. Bevorzugt sind Betamethason, Thioctsäure, Sotalol, Salbutamol, Norfenefrin, Silymarin, Dihydroergotamin, Buflomedil, Etofibrat, Indometacin, Oxazepam, beta-Acetyldigoxim, Piroxicam, Haloperidol, ISMN, Amitriptylin, Diclofenac, Nifedipin, Verapamil, Pyritinol, Nitrendipin, Doxycyclin, Bromhexin, Methylprednisolon, Clonidin, Fenofibrat, Allopurinol, Pirenzepin, Levothyroxin, Tamoxifen, Metildigoxin, o-(beta-Hydroxyethyl)rutosid, Propicillin, Aciclovir-mononitrat, Paracetamol, Naftidrofuryl, Pentoxyfyllin, Propafenon, Acebutolol, L-Thyroxin, Tramadol, Bromocriptin, Loperamid, Ketotifen, Fenoterol, Ca-Dobelisat, Propranolol, Minocyclin, Nicergolin, Ambroxol, Metoprolol, beta-Sitosterin, Enalaprilhydrogenmaleat, Benzafibrat, ISDN, Gallopamil, Xantinolnicotinat, Digitoxin, Flunitrazepan, Bencyclan, Dexapanthenol, Pindolol, Lorazepam, Diltiazem, Piracetam, Phenoxymethylpenicillin, Furosemid, Bromazepam, Flunarizin, Erythromycin, Metoclopramid, Acemetacin, Ranitidin, Biperiden, Metamizol, Doxepin, Dikalium-Chlorazepat, Tetrazepam, Estramustinphosphat, Terbutalin, Captopril, Maprotilin, Prazosin, Atenolol, Glibenclamid, Cefaclor, Etilefrin, Cimetidin, Theophyllin, Hydromorphon, Ibuprofen, Primidon, Clobazam, Oxaceprol, Medroxyprogesteron, Flecainid, Mg-Pyridoxal-5-phosphatglutaminat, Hymechromon, Etofyllinclofibrat, Vincamin, Cinnarizin, Diazepam, Ketoprofen, Flupentixol, Molsidomin, Glibornurid, Dimetinden, Melperon, Soquinolol, Dihydrocodein, Clomethiazol, Clemastin, Glisoxepid, Kallidinogenase, Oxyfedrin, Baclofen, Carboxymethylcystein, Thioridacin, Betahistin, L-Tryptophan, Myrtol, Bromalaine, Prenylamin, Salazosulfa pyridin, Astemizol, Sulpirid, Benzerazid, Dibenzepin, Acetylsalicylsäure, Miconazol, Nystatin, Ketoconazol, Na-Picosulfat, Colestyramin, Gemfibrocil, Rifampicin, Fluorocortolon, Mexiletin, Amoxicillin, Terfenadrin, Mucopolysaccharidpolyschwefelsäureester, Triazolam, Mianserin, Tiaprofensäure, Ameziniummetilsulfat, Mefloquin, Probucol, Chinidin, Carbamepin, Mg-L-aspartat, Penbutolol, Piretanid, Amitriptylin, Cyproteron, Na-Valpropinat, Mebeverin, Bisacodyl, 5-Amino-Salicylsäure, Dihydralazin, Magaldrat, Phenprocoumon, Amantadin, Naproxen, Carteolol, Famotidin, Methyldopa, Auranofin, Estriol, Nadolol, Levomepromazin, Doxorubicin, Medofenoxat, Azathioprin, Flutamid, Norfloxacin, Fendilin, Prajmaliumbitartrat, Aescin.

Besonders bevorzugt werden feste Lösungen folgender Wirkstoffe: Acetaminophen (= Paracetamol), Acetohexamid, Acetyldigoxim, Acetylsalicylsäure, Acromycin, Anipamil, Benzocain, beta-Carotin, Chloramphenicol, Chlordiazepoxid, Chlormadinoacetat, Chlorthiazid, Cinnarizin, Clonazepam, Codein, Dexamethason, Diazepam, Dicumarol, Digitoxin, Digoxin, Dihydroergotamin, Drotaverin, Flunitrazepam, Furosemid, Gramicidin, Griseofluvin, Hexobarbital, Hydrochlorothiazid, Hydrocortison, Hydroflumethazid, Indimethazin, Ketoprofen, Lonetil, Medazepam, Mefrusid, Methandrostenolon, Methylprednisolon, Methylsulfadiazin (= Sulfaperin), Nalidixinsäure, Nifedipin, Nitrazepam, Nitrofurantoin, Nystatin, Östradiol, Papaverin, Phenacetin, Phenobarbital, Phenylbutazon, Phenytoin, Prednison, Reserpin, Spironolacton, Streptomycin, Sulfadimidin (= Sulfamethazin), Sulfamethizol, Sulfamethoxazol (= Sulfameter), Sulfaperin, Sulfathiazol, Sulfisoxazol, Testosteron, Tolazamid, Tolbutamid, Trimethoprim, Tyrothricin.

Der Begriff "feste Lösungen" ist dem Fachmann geläufig, siehe dazu Chiou und Riegelman, J. Pharm. Sci. 60, 1281-1302 (1971). In festen Lösungen von Wirkstoffen in Polymeren liegt der Wirkstoff molekulardispers verteilt in der Matrix vor.

Das in Wasser und gastrointestinalen Flüssigkeiten unlösliche natürliche, halbsynthetische oder synthetische Polymer b) kann z.B. ein Celluloseether wie Ethylcellulose oder ein Celluloseester wie Cellulose-diacetat, Cellulosetriacetat, Celluloseacetat-propionat und Celluloseacetat-butyrat sein. Darüber hinaus lassen sich auch unlösliche Polysaccharide wie Chitin und Chitinderivate und mikrokristalline Cellulose einsetzen. Beispiele geeigneter synthetischer Polymerer sind Poly(meth)acrylsäureester, Homo- und Copolymere des Vinylacetats u.ä. Bevorzugt sind Ethylcellulosen.

Die wasserunlösliche lipophile Komponente c) mit weichmachenden Eigenschaften bezüglich des Polymeren b) und Gleit- bzw. Schmiermitteleigenschaften kann z.B. ein Fettalkohol wie Cetyl- oder Stearylalkohol, eine Fettsäure wie Stearinsäure oder ein Wachs, beispielsweise Esterwachs auf Basis von Montanwachs, sein. Erfindungsgemäß verwendbar sind ferner z.B. polyoxethylierte Fettalkohole, Fettsäuren und Pflanzenöle, hydrierte Pflanzenöle, Mono-, Di- und Triglyceride sowie Lecithine. Darüber hinaus sind Polyglycerinfettsäureester, gesättigte polyoxethylierte Glyceride, Polyethylenoxide, Polypropylenoxide bzw. deren Blockcopolymere, Phthalsäureester, acetylierte Monoglyceride einsetzbar. Bevorzugt sind Mono-, Di- oder Triglyceride oder deren Gemische und Polyglycerinfettsäureester. Bevorzugt sind lipophile Komponenten c) mit einem HLB (hydrophilic/lipophilic balance)-Wert von 1 bis 9, insbesondere 2 bis 5.

Als Gelbildner d), also in Wasser hochviskose kolloidale Lösungen oder Gele bildende oder in Wasser zumindest quellende Polymere, kommen insbesondere wasserlösliche Cellulosederivate in Betracht wie Alkyl-cellulosen, Hydroxyalkylcellulosen, Hydroxyalkyl-allylcellulosen, z.B. Methylcellulose, Hydroxymethylcellulose, Hydroxyethylcellulose, Hydroxypropylcellulose, Hydroxybutylcellulose, Hydroxyethylmethylcellulose, Hydroxypropylmethylcellulose; ferner Carboxyalkylcellulosen, Carboxyalkyl-alkylcellulosen, Carboxyalkylcelluloseester, z.B. Carboxymethylcellulose und ihre Alkalisalze; sie können auch andere wasserlösliche Polysaccharide sein, wie Alginsäuren und ihre Salze (Alginate), Carrageenane, Guargummi, Xanthan-Gummi, Agar-Agar, Gummi arabicum und verwandte Gummen, Pectine, Galactomannane, Traganth, ferner wasserlösliche Chitin-Derivate wie Chitosan. Bevorzugt sind wasserlösliche Alkylcellulosen, Hydroxyalkylcellulosen oder Hydroxyalkyl-alkylcellulosen, die als 2%ige Lösung in Wasser bei 20°C eine Viskosität über 1000 cps, vorzugsweise von 3500 bis 120 000 cps, aufweisen. Ganz besonders bevorzugt sind Hydroxypropylmethylcellulosen mit einem Methylierungsgrad von 1.36 bis 1.81 und einem Hydroxypropylierungsgrad von 0.12 bis 0.23, sowie Hydroxypropylcellulosen.

Synergistische Viskositätsanstiege durch Mischungen der Polymerkomponenten, beispielsweise Hydroxypropylcellulosen mit anionischen Polymeren wie Carboxymethylcellulosen oder Natriumalginat, sind besonders vorteilhaft.

"Wasserlöslich" heißt, daß sich in 100 Gramm Wasser von 20°C mindestens 0,5, vorzugsweise mindestens 2 Gramm des Polymeren kolloidal lösen.

) Als Polymerkomponente d) kommen auch in Wasser bzw. intestinalen Flüssigkeiten unlösliche, aber hydrophile quellbare Polymere wie vernetztes Polyvinylpyrrolidon oder vernetzte Stärken, Stärkederivate wie Natrium-Stärkeglycolat, Croscarmellose-Natrium, niedrig substituierte Hydroxylpropylcellulose und niedrig substituierte vernetzte Natriumcarboxymethylcellulose in Frage.

Entscheidend für die Eignung eines Polymeren als Komponente d) ist, daß es einerseits hydrophil ist, andererseits nicht zu schnell im Verdauungstrakt in Lösung geht. Es soll einerseits das Herausdiffundieren des Wirkstoffs aus dem Innern der Pellets ermöglichen, andererseits soll dies nur langsam geschehen. Deshalb soll es mit Wasser ein Gel oder eine hochviskose Lösung bilden. Die Wahl dieser Komponente und ihrer Menge hat entscheidenden Einfluß auf den Retardierungseffekt. Es hat sich überraschend gezeigt, daß die o.g. natürlichen oder halbsynthetischen hydrophilen, gelbildenden Polymeren - im Gegensatz zu vollsynthetischen Polymeren wie PVP oder Vinylpyrrolidon-Vinylacetat-Copolymere - hohe Lagerstabilität (Konstanz des Retardeffektes beim Lagern) gewährleisten.

Die Komponente e) kann aus einem oder mehreren der für derartige Zwecke üblichen Hilfsstoffe bestehen, wie Füllstoffe, Schmiermittel, Formentrennmittel, Weichmacher, Treibmittel, Stabilisatoren, Farbstoffe, Streckmittel, Fließmittel sowie deren Mischungen. Beispiele für Füllstoffe sind anorganische Füllstoffe wie die Oxide von Magnesium, Aluminium, Silizium, Titan etc. sowie mikrokrist. Cellulose und Cellulosepulver, verschiedene Stärken und deren Abbauprodukte (Maltodextrine), Lactose, Mannit, Calciumdiphosphat in einer Konzentration von 0.02 bis 50, vorzugsweise von 0,20 bis 20 Gew.%, bezogen auf das Gesamtgewicht der Pellets.

Beispiele für Schmiermittel sind Stearate von Aluminium und Calcium sowie Talkum und Silicone in einer Konzentration von 0,1 bis 5, vorzugsweise von 0,1 bis 3 Gew.-%, bezogen auf das Gesamtgewicht der Pellets.

Beispiele für Weichmacher beinhalten niedermolekulare Poly-(alkylenoxide), wie Poly(ethylenglycole), Poly(propylenglycole), Poly(ethylen-propylenglycole); organische Weichmacher mit niederem Molekulargewicht wie Glycerin, Pentaerythrit, GlycerinMonoacetat, -Diacetat oder -Triacetat, Propylenglycol, Sorbit, Natriumdiethylsulfonsuccinat, zugefügt in Konzentrationen von 0,5 bis 15, vorzugsweise von 0,5 bis 5 Gew.%, bezogen auf das Gesamtgewicht der Pellets.

Beispiele für Farbstoffe sind bekannte Azofarbstoffe, organische und anorganische Pigmente, oder Farbmittel natürlicher Herkunft. Anorganische Pigmente sind bevorzugt, in Konzentrationen von 0,001 bis 10, vorzugsweise von 0,5 bis 3 Gew.-%, bezogen auf das Gesamtgewicht der Pellets.

Unter Hilfsstoffen im Sinne der Erfindung sind auch Substanzen zur Herstellung einer festen Lösung mit dem pharmazeutischen Wirkstoff zu verstehen. Diese Hilfsstoffe sind beispielsweise Zucker und Zuckeralkohole wie Mannit, Sorbit, Xylit, ferner Harnstoff, Pentaerythrit und Pentaerythrit-tetraacetat, Polymere wie Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z.B. bei J.L. Ford, Pharm. Acta Helv. 61, 69-88 (1986) angegeben.

Als pharmazeutische Hilfsstoffe gelten auch Zusätze von Basen oder Säuren zur Steuerung der Löslichkeit eines Wirkstoffes (siehe z.B. K. Thoma et al., Pharm. Ind. 51, 98-101 (1989)).

Das Mischen des Wirkstoffs oder der Wirkstoffe mit den polymeren Bindemitteln und gegebenenfalls weiteren üblichen galenischen Zusätzen kann vor oder nach dem Schmelzen des polymeren Bindemittels nach den in der Technik üblichen Verfahren erfolgen. Bevorzugt wird das Mischen im Extruder, vorzugsweise einem Zweischneckenextruder oder einem Einschneckenextruder mit Mischabteil.

Das polymere Bindemittelgemisch soll in der Gesamtmischung aller Komponenten im Bereich von 50 bis 200, vorzugsweise 50 bis 180, insbesondere 60 bis 150°C erweichen oder schmelzen, so daß die Masse extrudierbar ist.

Die Schmelzen sind lösungsmittelfrei. Damit ist gemeint, daß kein Wasser und kein organisches Lösungsmittel zugesetzt wird.

Die Formgebung erfolgt durch Schmelz-Extrusion bei 50 bis 200, vorzugsweise 50 bis 180, insbesondere 60 bis 150°C und anschließende kontinuierliche Verformung des noch plastischen Stranges, z.B. durch Verformen zu Tabletten, beispielsweise gemäß EP-A 240 906 durch Hindurchführen des Stranges zwischen zwei gegenläufig angetriebenen Walzen mit einander gegenüberliegenden Vertiefungen im Walzenmantel, deren Ausführung die Tablettenform bestimmt. Auch Kaltabschlag kommt in Betracht.

Bevorzugt wird der sogenannte Heißabschlag. Dabei werden die Stränge unmittelbar nach dem Austritt aus der Düsenanordnung am Extruder durch beispielsweise rotierende Messer oder eine andere geeignete Anordnung zerkleinert, zweckmäßig in Stücke, deren Länge etwa gleich dem Strangdurchmesser ist. Diese abgeschlagenen Schmelzeteilchen kühlen im Luft- oder Gasstrom so weit ab, daß die Oberfläche vor einer Berührung mit anderen Teilchen oder einer Gefäßwand bereits klebfrei ist, andererseits die Teilchen aber noch so plastisch sind, daß sie durch Zusammenstöße, z.B. mit der Wandung eines angeschlossenen Cyclons, eine sphärische Form annehmen. Man erhält so in einfacher Weise weitgehend kugel- oder linsenförmige Teilchen mit Durchmessern von 0,5 bis 4, vorzugsweise 0,8 bis 2 mm. Die bevorzugten kleineren Teilchen sind in erster Linie zum Füllen von Kapseln geeignet.

Die Erfindung gestattet in einfacher und umweltfreundlicher Weise (ohne Lösungsmittel) die Herstellung von Retard-Matrixpellets, die in weiten Grenzen hinsichtlich ihrer Wirkstofffreisetzung steuerbar sind. Die verzögerte Wirkstofffreisetzung gelingt ohne Aufbringung eines steuernden Filmüberzugs, der aus organischen Lösungsmitteln oder wäßrigen Dispersionen appliziert werden müßte und einen Trocknungsschritt verlangen würde. Die Wirkstofffreisetzung erfolgt nach Erosions- und Diffusionskontrolle. Die Erfindung eröffnet die Möglichkeit, auch eine pH-unabhängige Wirkstofffreisetzung zu erzielen. Die erfindungsgemäßen Formen sind für Wirkstoffe mit sehr unterschiedlichen Lösungseigenschaften geeignet. Der Retardeffekt kann auch bei kleinen Retardformen außerordentlich stark eingestellt werden. Das Verfahren gestattet die Herstellung fester Lösungen der Wirkstoffe im Matrixpolymeren durch die Schmelztechnologie ohne Verwendung organischer Lösungsmittel. Feste Lösungen zeichnen sich durch verbesserte Bioverfügbarkeit aus. Das Verfahren ist sehr wirtschaftlich, weil kontinuierlich, es ist dadurch traditionellen Pelletierverfahren überlegen. Die erfindungsgemäßen Pellets können einen hohen Wirkstoffanteil aufweisen. Die Streuung der Wirkstofffreisetzung ist aufgrund der guten Homogenität der Massen gering und hervorragend reproduzierbar. Die Kinetik der Wirkstofffreisetzung bleibt auch unter extremen klimatischen Lagerbedingungen (mindestens 1 Monat Lagerung bei 50°C bzw. bei 30°C und 75 % relativer Luftfeuchtigkeit) überraschend stabil (Abweichung in der Freisetzung max. 20 % absolut, vgl. Tabelle II).

Der Vorteil des Extrusionsverfahrens gegenüber anderen Techniken wie Granulieren und Tablettieren besteht in der einfachen Technologie, der Vermeidung von Lösungsmitteln, minimierter Anzahl und Menge an Hilfsstoffen, der Möglichkeit der Herstellung von festen Lösungen, der Vermeidung von Möglichkeiten zur Entmischung der Komponenten, mit anderen Worten in einer zuverlässig gleichmäßigen Zusammensetzung der einzelnen Retardformen während der gesamten Produktion. Hinzu kommen die Vorteile eines kontinuierlichen Verfahrensablaufes mit hohem Durchsatz bei geringen Materialverlusten.

Beispiele

Die in der Tabelle angegebenen Gewichtsteile an Wirkstoff, Polymeren und lipophiler Komponente und anderen Hilfsstoffen wurden entweder vorgemischt oder über separate Dosierwaagen direkt in den Einzug eines Doppelschnecken-Extruders (Werner & Pfleiderer, ZSK 30) eingetragen. Die Schmelzextrusion erfolgte mit einem Produktdurchsatz von ca. 3 bis 4 kg/h. Die Temperaturen der einzelnen Temperaturzonen ("Schüsse") des Extruders waren 30/150/100/100/100 °C, die Temperatur der beheizten Düsenleiste ist in der Tabelle separat angegeben. Die Düsenleiste wies 7 Bohrungen á 1 mm Durchmesser auf. Die über die beheizte Extruderdüsenleiste austretenden Schmelzstränge wurden durch luftgekühlten Heißabschlag mit einem Messerwalzengranulator pelletiert.

Die Wirkstoff-Freisetzung wurde mittels der Rührflügelmethode (Paddle Methode nach USP XXI) gemessen. Diese in-vitro-Prüfmethode dient zur Bestimmung der Lösungsrate von wirkstoffhaltigen Formlingen, z.B. Tabletten.

Hierzu wurden 900 ml eines Phosphatpuffers mit einem pH-Wert von 6,8 in einem 1 l-Gefäß mit Rundboden auf 37°C temperiert. Eine geeignete Menge an Pellets (ca. 300 mg) der Korngröße 1,25 bis 1,60 mm wurde eingewogen. Die Wirkstoff-Freisetzung der Pellets in Prozent wurde in diesem No-Change-Test nach USP XXI bei einer Paddle-Drehzahl von 100 Upm nach jeweils 1, 2, 3, 4, 5, 6, 7 und 8 Stunden UV-spektroskopsich bestimmt.

Tabelle I

Als Wirkstoff wurden bei den Beispielen 1 bis 39 je 50 Gew.-% Gallopamil-Hydrochlorid, bei den Beispielen 40 bis 65 je 50 Gew.-% Theophyllin eingesetzt.

| Bsp. Nr. | unlösliches Polymer b) | % | lipophile Komponente c) | % | Gelbildner d) | % | Düsen-temp. [°C] | Freisetzungsprofil (% nach 1 bis 8 h) |
|---|---|---|---|---|---|---|---|---|
| 1 | Ethocel® N 7 | 15 | Precirol® Ato 5 | 10 | Klucel® HF | 25 | 120 | 53/71/81/87/90/93/95/96 |
| 2 | dito | 20 | dito | 10 | dito | 20 | 122 | 59/76/85/90/93/94/95/97 |
| 3 | dito | 25 | dito | 10 | dito | 15 | 122 | 59/76/85/90/93/94/96/96 |
| 4 | dito | 30 | dito | 10 | dito | 10 | 125 | 62/79/87/92/94/96/97/97 |
| 5 | dito | 35 | dito | 10 | dito | 5 | 124 | 63/77/84/88/90/92/94/95 |
| 6 | dito | 10 | dito | 15 | dito | 25 | 125 | 52/70/80/85/89/93/95/96 |
| 7 | dito | 15 | dito | 15 | dito | 20 | 116 | 63/80/88/93/96/98/99/100 |
| 8 | dito | 20 | dito | 15 | dito | 15 | 110 | 54/73/81/86/90/92/94/96 |
| 9 | dito | 25 | dito | 15 | dito | 10 | 115 | 48/66/74/78/82/84/86/87 |
| 10 | dito | 30 | dito | 15 | dito | 5 | 104 | 31/43/49/54/57/59/61/63 |
| 11 | dito | 10 | dito | 20 | dito | 20 | 65 | 38/55/66/73/78/82/85/87 |
| 12 | dito | 15 | dito | 20 | dito | 15 | 67 | 32/46/56/62/67/71/74/76 |

EP 0 756 480 B1

| Bsp. Nr. | unlösliches Polymer b) | % | lipophile Komponente c) | % | Gelbildner d) | % | Düsentemp. [°C] | Freisetzungsprofil (% nach 1 bis 8 h) |
|---|---|---|---|---|---|---|---|---|
| 13 | dito | 20 | dito | 20 | dito | 10 | 88 | 28/39/46/51/54/57/59/61 |
| 14 | dito | 25 | dito | 20 | dito | 5 | 87 | 21/28/32/36/38/39/41/42 |
| 15 | dito | 10 | dito | 25 | dito | 15 | 65 | 29/43/51/57/61/64/67/79 |
| 16 | dito | 15 | dito | 25 | dito | 10 | 61 | 23/33/40/45/48/50/52/55 |
| 17 | dito | 20 | dito | 25 | dito | 5 | 59 | 17/23/27/30/33/34/35/38 |
| 18 | dito | 10 | dito | 30 | dito | 10 | 58 | 23/34/41/46/50/53/55/57 |
| 19 | dito | 15 | dito | 30 | dito | 5 | 59 | 22/32/39/43/56/48/49/51 |
| 20 | dito | 10 | dito | 35 | dito | 5 | 53 | 16/22/26/29/31/32/34/36 |
| 21 | dito | 10 | handelsübliche höherkettige Partialglyceride | 20 | Klucel EF | 20 | 90 | 36/49/56/61/65/68/71/73 |
| 22 | dito | 15 | dito | 20 | dito | 15 | 88 | 34/45/51/55/58/60/63/65 |
| 23 | dito | 20 | dito | 20 | dito | 10 | 88 | 31/39/44/47/49/51/53/55 |
| 24 | dito | 25 | dito | 20 | dito | 5 | 84 | 22/26/28/30/31/32/33/33 |
| 25 | Avicel PH 101 | 20 | dito | 20 | Klucel HF | 10 | 118 | 69/88/96/100 |
| 26 | Ethylcellulose Typ NF 100 | 10 | dito | 20 | dito | 20 | 109 | 39/56/65/71/76/79/82/84 |
| 27 | dito | 15 | dito | 20 | dito | 15 | 110 | 24/37/46/52/60/62/64 |
| 28 | dito | 20 | dito | 20 | dito | 10 | 110 | 20/27/32/34/37/38/40/41 |
| 29 | dito | 25 | dito | 20 | dito | 5 | 110 | 15/20/22/23/24/25/26/28 |

EP 0 756 480 B1

| Bsp. Nr. | unlösliches Polymer b) | % | lipophile Komponente c) | % | Gelbildner d) | % | Düsen-temp. [°C] | Freisetzungsprofil (% nach 1 bis 8 h) |
|---|---|---|---|---|---|---|---|---|
| 30 | Ethylcellulose Typ NF 7 | 20 | Polyglyceryl-3-Distearat | 20 | dito | 10 | 100 | 48/68/77/83/87/90/92/93 |
| 31 | dito | 10 | Glycerin-behenat | 20 | dito | 20 | 100 | 33/54/67/76/83/87/92/94 |
| 32 | dito | 15 | dito | 20 | dito | 15 | 100 | 32/50/63/72/78/82/86/89 |
| 33 | dito | 20 | dito | 20 | dito | 10 | 103 | 34/49/58/65/69/73/76/78 |
| 34 | dito | 25 | dito | 20 | dito | 5 | 103 | 29/40/47/52/56/59/62/63 |
| 35 | dito | 20 | Polyglycerin-palmitostearat | 20 | dito | 10 | 100 | 65/80/89/94/96/97/100/100 |
| 36 | dito | 25 | Polyglycolisier-tes natürliches Wachs | 20 | dito | 5 | 98 | 53/68/77/83/86/89/91/93 |
| 37 | dito | 10 | Hydriertes Ricinusöl | 20 | dito | 15 | 80 | 33/54/66/75/80/83/86/88 |
| 38 | dito | 20 | dito | 20 | dito | 10 | 85 | 30/47/59/67/73/76/79/82 |
| 39 | dito | 25 | dito | 20 | dito | 5 | 90 | 30/44/56/63/68/71/74/75 |
| 40 | Ethocel N7 | 10 | Precirol Ato5 | 20 | Klucel HF | 20 | 85 | 0/28/41/49/55/60/65/68/70 |
| 41 | dito | 15 | dito | 20 | dito | 15 | 80 | 0/27/41/49/56/61/65/69/72 |
| 42 | dito | 20 | dito | 20 | dito | 10 | 80 | 0/27/42/52/59/65/70/74/77 |
| 43 | dito | 25 | dito | 20 | dito | 5 | 80 | 0/24/36/44/49/54/58/62/65 |
| 44 | dito | 10 | dito | 20 | Klucel EF | 20 | 80 | 0/53/74/85/92/95/98/100/100 |
| 45 | dito | 15 | dito | 20 | dito | 15 | 85 | 0/45/61/72/79/85/89/92/93 |

EP 0 756 480 B1

| Bsp. Nr. | unlösliches Polymer b) | % | lipophile Komponente c) | % | Gelbildner d) | % | Düsentemp. [°C] | Freisetzungsprofil (% nach 1 bis 8 h) |
|---|---|---|---|---|---|---|---|---|
| 46 | dito | 20 | dito | 20 | dito | 10 | 85 | 0/26/35/42/47/51/54/58/61 |
| 47 | dito | 25 | dito | 20 | dito | 5 | 80 | 0/17/23/27/30/32/34/36/38 |
| 48 | dito | 15 | dito | 25 | Klucel HF | 10 | 60 | 0/14/19/23/26/28/31/33/35 |
| 49 | dito | 20 | dito | 25 | dito | 5 | 60 | 0/15/20/22/25/28/29/32/34 |
| 50 | Ethocel N7 | 15 | Precirol Ato5 | 30 | dito | 5 | 60 | 0/9/13/15/17/19/21/23/25 |
| 51 | Ethocel NF100 | 10 | dito | 20 | Klucel EF | 20 | 80 | 0/27/42/51/58/65/68/74/77 |
| 52 | dito | 15 | dito | 20 | dito | 15 | 89 | 0/18/27/32/36/41/43/47/50 |
| 53 | dito | 20 | dito | 20 | dito | 10 | 100 | 0/12/17/20/22/24/25/27/28 |
| 54 | dito | 25 | dito | 20 | dito | 5 | 115 | 0/6/8/9/10/11/11/12/12 |
| 55 | dito | 10 | dito | 25 | dito | 15 | 88 | 0/17/27/33/38/41/45/45/50/51 |
| 56 | dito | 15 | dito | 25 | dito | 10 | 95 | 0/11/16/20/23/25/27/30/31 |
| 57 | dito | 20 | dito | 25 | dito | 5 | 100 | 0/7/10/11/13/14/15/16/16 |
| 58 | Ethocel N7 | 10 | hydriertes Ricinusöl | 20 | dito | 20 | 105 | 0/52/76/88/93/95/97/98/99 |
| 59 | dito | 15 | dito | 20 | dito | 15 | 90 | 0/40/56/65/73/79/83/88/89 |
| 60 | dito | 20 | dito | 20 | dito | 10 | 87 | 0/27/39/48/54/59/64/69/72 |
| 61 | dito | 25 | dito | 20 | dito | 5 | 85 | 0/14/20/24/28/32/35/38/41 |
| 62 | Ethocel NF100 | 10 | dito | 20 | dito | 20 | 99 | 0/39/59/74/82/90/93/97/100 |
| 63 | dito | 15 | dito | 20 | dito | 15 | 100 | 0/23/37/47/54/61/67/72/75 |

EP 0 756 480 B1

| Bsp. Nr. | unlösliches Polymer b) | % | lipophile Komponente c) | % | Gelbildner d) | % | Düsen- temp. [°C] | Freisetzungsprofil (% nach 1 bis 8 h) |
|---|---|---|---|---|---|---|---|---|
| 64 | dito | 20 | dito | 20 | dito | 10 | 110 | 0/13/18/24/29/32/35/38/40 |
| 65 | dito | 25 | dito | 20 | dito | 5 | 117 | 0/8/10/13/15/16/17/18/19 |

Ethocel® =      Ethylcellulose der Firma Dow, USA

Precirol® Ato5 =      höherkettiges Partialglycerid der Fa. Gattefossé, Frankreich

Klucel® =      Hydroxypropylcellulose der Firma Hercules, USA

Avicel =      Mikrokristalline Cellulose der Firma FMC, USA

EP 0 756 480 B1

Tabelle II

Beispiele zur Lagerstabilität der erfindungsgemäßen Formen

| Bsp. Nr. | Freisetzung (% in 1 bis 8 h) der Formen nach Lagerung (1 Monat bei 50°C) | Freisetzung (% nach 1 bis 8 h) der Formen nach Lagerung (1 Monat bei 30°C 75% rel. Feuchte) |
|---|---|---|
| 21 | 26/46/58/65/71/74/77/80 | 25/37/45/51/56/60/63/65 |
| 22 | 28/42/52/58/63/67/71/73 | 29/38/44/48/51/54/56/58 |
| 23 | 21/32/39/44/48/51/53/55 | 26/34/41/43/45/47/48 |
| 24 | 15/20/23/25/27/29/31/32 | 22/27/29/30/31/32/33/34 |
| | | |
| 33 | 29/50/63/72/77/81/84/86 | 34/48/58/64/70/72/75/77 |
| 34 | 28/45/55/61/67/70/74/76 | 27/37/43/47/50/53/56/57 |
| 37 | 27/44/56/64/70/76/78/80 | 26/40/50/58/63/67/71/74 |
| 38 | 27/45/58/67/74/77/81/83 | 28/43/55/64/69/73/76/78 |
| 39 | 28/48/61/70/75/78/81/83 | 27/40/50/57/61/64/67/69 |

Tabelle III

Vergleichsversuche zur unvollständigen Freisetzung aus inert-hydrophober Matrix <u>ohne</u> Gelbildner

| Bsp. Nr. | Wirkstoff | | unlösliches Polymer | % | lipophile Komponente | % | Düsen-temp. [°C] | Freisetzungsprofil (% nach 1 bis 8 h) |
|---|---|---|---|---|---|---|---|---|
| 2 | Gallopamil-Hydrochlorid | 50 | Ethylcellulose Typ NF7 | | Precirol Ato5 | 15 | 107 | 18/22/25/27/29/30/31/32 |
| 3 | dito | 50 | dito | 35 | dito | 20 | 87 | 15/19/21/22/23/24/25/25 |
| 4 | dito | 50 | dito | 30 | dito | 25 | 74 | 17/21/25/27/29/30/31/32 |
| 5 | dito | 50 | dito | 25 | dito | 30 | 56 | 19/26/32/35/37/38/40/40 |
| 6 | dito | 50 | dito | 20 | dito | 35 | 57 | 15/21/26/29/30/31/33/33 |
| 7 | dito | 50 | dito | 15 | hydriertes Ricinusöl | 20 | 95 | 19/28/35/41/45/48/51/53 |
| 8 | dito | 50 | Ethylcellulose Typ NF100 | 30 | Precirol Ato5 | 20 | 112 | 16/20/22/24/25/26/27 |
| 9 | dito | 50 | Ethylcellulose Typ NF7 | 30 | Glycerinbehenat | 20 | 103 | 25/33/37/40/42/44/46/47 |
| 10 | Theophyllin | 50 | dito | 30 | Precirol Ato5 | 20 | 80 | 11/14/16/17/18/19/20/21 |
| 11 | dito | 50 | Ethylcellulose Typ NF100 | 30 | dito | 20 | 125 | 3/4/4/5/5/5/5/6 |
| 12 | dito | 25 | dito | 25 | dito | 25 | 100 | 6/7/9/9/10/10/11/11 |

EP 0 756 480 B1

EP 0 756 480 B1

| Bsp. Nr. | Wirkstoff | | unlösliches Polymer | % | lipophile Komponente | % | Düsen-temp. [°C] | Freisetzungsprofil (% nach 1 bis 8 h) |
|---|---|---|---|---|---|---|---|---|
| 13 | dito | 50 | dito | 30 | hydriertes Ricinusöl | 20 | 140 | 2/3/3/3/4/4/4/4 |
| 14 | dito | 50 | Ethylcellulose Typ NF7 | 30 | dito | 20 | 95 | 4/5/5/6/7/7/7/8 |

Tabelle IV

Vergleichsversuche zur Lagerstabilität (gemäß EP-B 0 024 596)

| Bsp. Nr. | Wirkstoff | % | unlösliches Polymer | % | lipophile Komponente | % | Nicht hydrophiles Polymer II | | Freisetzungsprofil (% nach 1 bis 8 h)<br><br>A Original nach Herstellung<br>B Lagerung 1 Monat bei 50°C |
|---|---|---|---|---|---|---|---|---|---|
| 1 | Gallopamil Hydrochlorid | 50 | Ethylcellulose Typ NF7 | 10 | Precirol Ato5 | 20 | Vinylpyrrolidon-Vinylacetat-Copolymer (Kollidon® VA64) | 20 | A<br>45/60/67/72/76/78/81/83<br>B<br>52/69/76/81/84/86/88/90 |
| 2 | dito | 50 | dito | 15 | dito | 20 | dito | 15 | A<br>35/47/52/56/60/63/66/68<br>B<br>41/62/72/76/79/81/83/84 |
| 3 | dito | 50 | dito | 20 | dito | 20 | dito | 10 | A<br>27/35/39/42/44/45/47/48<br>B<br>28/44/54/61/67/70/73/75 |
| 4 | dito | 50 | dito | 25 | | 20 | dito | 5 | A<br>22/28/31/33/34/35/36/36<br>B<br>14/24/32/38/43/47/51/54 |

EP 0 756 480 B1

**Patentansprüche**

1. Retard-Matrixpellets mit kugel- bis linsenförmiger Gestalt und einheitlichen größten Durchmessern im Bereich von 0,5 bis 4 mm, bestehend aus

   a) 0,1 bis 87 Gew.-% mindestens einer biologisch wirksamen Verbindung,

   b) 5 bis 50 Gew.-% mindestens eines wasserunlöslichen Polymeren,

   c) 5 bis 45 Gew.-% mindestens einer lipophilen Komponente als Weichmacher für das Polymer b),

   d) 3 bis 40 Gew.-% eines natürlichen oder halbsynthetischen Gelbildners,

   e) 0 bis 50 Gew.-% eines oder mehrerer üblicher Formulierungshilfsmittel.

2. Retard-Matrixpellets nach Anspruch 1, gekennzeichnet durch folgende Konzentrationen der Komponenten:

   a) 1 bis 75 Gew.-%,

   b) 10 bis 40 Gew.-%,

   c) 10 bis 35 Gew.-%,

   d) 5 bis 25 Gew.-%,

   e) 0 bis 40 Gew.-%.

3. Retard-Matrixpellets nach Anspruch 1 oder 2, **dadurch gekennzeichnet**, daß der größte Durchmesser 0,8 bis 2 mm beträgt.

4. Retard-Matrixpellets nach einem der Ansprüche 1 bis 3, **dadurch gekennzeichnet**, daß der Wirkstoff (a) pharmazeutischer Natur ist.

5. Kontinuierliches, einstufiges Verfahren zur Herstellung von Retard-Matrixpellets nach einem der Ansprüche 1 bis 4 durch Extrusion der geschmolzenen Mischung der Komponenten bei 50 bis 200° und kontinuierliche Formgebung.

6. Verfahren nach Anspruch 5, **dadurch gekennzeichnet**, daß bei 50 bis 180°C extrudiert wird.

7. Verfahren nach Anspruch 5, **dadurch gekennzeichnet**, daß bei 60 bis 150°C extrudiert wird.

8. Verfahren nach einem der Ansprüche 5 bis 7, **dadurch gekennzeichnet**, daß die Formgebung durch Heißabschlag erfolgt.


**Claims**

1. Slow-release matrix pellets with a spherical or lenticular shape and uniform maximum diameters in the range from 0.5 to 4 mm, composed of

   a) 0.1-87 % by weight of at least one biologically active compound,

   b) 5-50 % by weight of at least one water-insoluble polymer,

   c) 5-45 % by weight of at least one lipophilic component as plasticizer for polymer b),

   d) 3-40 % by weight of a natural or semisynthetic gel former,

e) 0-50 % by weight of one or more conventional formulation aids.

**2.** Slow-release matrix pellets as claimed in claim 1, having the following concentrations of the components:

   a) 1-75 % by weight,

   b) 10-40 % by weight,

   c) 10-35 % by weight,

   d) 5-25 % by weight,

   e) 0-40 % by weight.

**3.** Slow-release matrix pellets as claimed in claim 1 or 2, wherein the maximum diameter is 0.8-2 mm.

**4.** Slow-release matrix pellets as claimed in any of claims 1 to 3, wherein the active substance (a) is pharmaceutical in nature.

**5.** A continuous one-stage process for producing slow-release matrix pellets as claimed in any of claims 1 to 4 by extrusion of the molten mixture of the components at 50-200°C and continuous shaping.

**6.** A process as claimed in claim 5, wherein extrusion is carried out at 50-180°C.

**7.** A process as claimed in claim 5, wherein extrusion is carried out at 60-150°C.

**8.** A process as claimed in any of claims 5 to 7, wherein hot-cut shaping takes place.


**Revendications**

**1.** Granulés à gangue à effet retard, de forme sphérique à lenticulaire, à grands diamètres uniformes dans l'intervalle de 0,5 à 4 mm, consistant en

   a) 0,1 à 87 % en poids d'au moins un composé possédant une activité biologique,
   b) 5 à 50 % en poids d'au moins un polymère dans l'eau,
   c) 5 à 45 % en poids d'au moins un composant lipophile, faisant fonction de plastifiant pour le polymère b),
   d) 3 à 40 % en poids d'un agent gélifiant naturel ou semi-synthétique,
   e) 0 à 50 % en poids d'un ou plusieurs auxiliaires de formulations usuels.

**2.** Granulés à gangue à effet retard selon la revendication 1, caractérisé par les concentrations suivantes des composants :

   a) 1 à 75 % en poids,
   b) 10 à 40 % en poids,
   c) 10 à 35 % en poids,
   d) 5 à 25 % en poids,
   e) 0 à 40 % en poids.

**3.** Granulés à gangue à effet retard selon la revendication 1 ou 2, caractérisé par le fait que le grand diamètre va de 0,8 à 2 mm.

**4.** Granulés à gangue à effet retard selon l'une des revendications 1 à 3, caractérisés par le fait que la substance active a) est de nature pharmaceutique.

**5.** Procédé continu à un seul stade opératoire pour la préparation des granulés à gangue à effet retard selon l'une des revendications 1 à 4, par extrusion du mélange des composants fondus à des températures de 50 à 200° et formage continu.

**6.** Procédé selon la revendication 5, caractérisé par le fait que l'on extrude à des températures de 50 à 180°C.

**7.** Procédé selon la revendication 5, caractérisé par le fait que l'on extrude à des températures de 60 à 150°C.

**8.** Procédé selon l'une des revendications 5 à 7, caractérisé par le fait que le formage est réalisé par découpage à chaud.